# FASCICULE DE BREVET EUROPEEN

(11) **EP 3 478 807 B1**
(45) Date de publication et mention de la délivrance du brevet: **15.04.2020**
(21) Numéro de dépôt: 17734338.1
(22) Date de dépôt: 30.06.2017
(51) Int. Cl.: C10M 171/02, C10M 101/04, C10G 32/02, C10G 71/02, C09F 7/04, A61K 8/92, A61Q 19/00, C11B 3/00

(54) **HUILE LUBRIFIANTE**
SCHMIERÖL
LUBRICATING OIL

(30) Priorité: 30.06.2016 BE 201605516
(43) Date de publication de la demande: 08.05.2019
(73) Titulaire: Green Frix, 7522 Blandain (BE)
(72) Inventeur: GODFROID, Thomas, 7110 Strépy-Bracquegnies (BE); POUSSARD, Loïc, 59860 Bruay sur l'Escaut (FR); POELMAN, Mireille, 7811 Arbre (BE); PEETERBROECK, Sophie, 1460 Virginal-Samme (BE); DANNEAUX, Frédéric, 1380 Lasne (BE); HOLVOET, Jean-Patrick, 7912 Saint-Sauveur (BE); MICHIELS, Matthieu, 7050 Herchies (BE)
(74) Mandataire: Calysta NV
(86) Numéro de dépôt international: PCT/EP2017/066369
(87) Numéro de publication internationale: WO 2018/002355

(56) Documents cités:
- FR-A- 808 648
- FR-A- 828 932
- FR-A- 828 933
- GB-A- 490 127
- US-A- 2 107 316

## Description

La présente invention se rapporte à une huile végétale telle que définie dans et par les revendications annexées, en particulier, une huile lubrifiante végétale, plus particulièrement une huile lubrifiante végétale par exemple traitée par décharges électriques.

Une huile lubrifiante végétale est connue par exemple du document antérieur FR808648 qui divulgue des matières organiques, par exemple une huile de Colza, modifiées par un traitement électrique (voltolisation). L'huile est alors traitée dans un récipient tournant dans lequel sont montées tour à tour des plaques de métal et des plaques de matière isolante. Un courant alternatif de basse fréquence (entre 50 Hz et 500 Hz) est alors appliqué entre les électrodes. Ce traitement permet d'obtenir des huiles végétales présentant une viscosité de l'ordre de E/50 = 20.

Le document FR828933 décrit la production d'huile ayant un indice de viscosité amélioré en mélangeant un agent d'addition et une huile minérale. L'agent d'addition est préparé en polymérisant une huile, de préférence une huile de Colza ou des huiles de graine de moutarde. Cette polymérisation est par exemple réalisée en soumettant l'huile à une décharge luminescente électrique alternative. Cette opération permet d'observer l'épaississement de l'huile jusqu'à une viscosité de maximum 1000 secondes Saybolt à 99°C et forme ainsi un polymère. L'étape suivante est alors la sulfuration du polymère ainsi obtenu. Le polymère sulfuré est alors ajouté à l'huile de base minérale dans laquelle ce polymère est destiné à être utilisé. L'huile ainsi obtenue par mélange du polymère et de l'huile minérale présente une viscosité d'environ 800 secondes Saybolt à 38 °C.

Dans le document GB490127, des huiles insaturées dont la viscosité est augmentée sont présentées. Toutefois, ce document enseigne un prétraitement consistant en l'hydrogénation sur un catalyseur au nickel de l'huile afin de réduire le nombre d'insaturation de celle-ci avant d'effectuer le traitement par décharges électriques. L'huile ainsi décrite, laquelle présente un nombre d'insaturations réduit dont l'indice d'iode est de l'ordre de 100, est alors soumise à un traitement par décharges électriques silencieuses afin de former une huile hydrogénée dont la viscosité est de l'ordre de 1000 secondes Saybolt à 210 °F. Cette huile hydrogénée est finalement mélangée avec une huile lubrifiante minérale (issue du pétrole), le mélange présentant des quantités égales d'huile hydrogénée et d'huile minérale. Ce mélange est alors à nouveau soumis à un traitement par décharges électriques afin de former un mélange d'huiles dont la viscosité est de l'ordre de 1000 secondes Saybolt à 210 °F.

Pour certaines applications, les propriétés physico-chimiques de l'huile lubrifiante utilisée sont particulièrement critiques pour atteindre de bonnes performances. Par exemple, les huiles de moteur sont généralement utilisées pour la lubrification des moteurs à explosion et permettent de lubrifier, nettoyer, inhiber la corrosion, améliorer l'étanchéité et contribuer à évacuer la chaleur de frictions et de combustion de façon à limiter la dégradation des pièces du moteur. En effet, les frottements produisent des particules de métal qui agissent comme un abrasif. L'huile permet de créer un film lubrifiant entre les surfaces en mouvement, minimisant le contact et donc l'abrasion qui est le principal facteur d'usure. Elle limite aussi l'oxydation et la corrosion du métal liée à l'oxygène encore présent dans le mélange gazeux. Bien entendu les huiles pour moteur sont soumises à des températures élevées, toutefois, la viscosité diminue avec l'augmentation de la température. Afin d'assurer son rôle, l'huile doit donc rester assez visqueuse pour maintenir son rôle de film protecteur, tout en restant assez fluide pour circuler librement dans le moteur, c'est pourquoi, il est indispensable que la viscosité de l'huile soit contrôlée et reproductible.

Dans d'autres applications telles que dans l'industrie alimentaire ou dans l'industrie cosmétique, il est crucial de contrôler la composition de l'huile lubrifiante pour des raisons de toxicité, notamment pour l'homme. En effet, les huiles minérales peuvent être issues de la distillation du pétrole et peuvent dès lors contenir des hydrocarbures aromatiques présentant une toxicité sévère. De plus, lorsque les huiles minérales sont soumises à un traitement par décharges électriques, les propriétés physico-chimiques de celles-ci sont modifiées par ce traitement et il n'est donc pas évident de contrôler les propriétés physico-chimiques du produit obtenu après ce traitement pas décharges électriques.

Il existe donc un besoin d'obtenir une huile végétale, par exemple lubrifiante qui présente des propriétés physico-chimiques contrôlables, contrôlées et reproductibles.

L'invention a pour but de pallier les inconvénients de l'état de la technique en procurant une huile végétale présentant une composition et des propriétés physico-chimiques contrôlables, contrôlées et reproductibles.

Pour résoudre ce problème, il est prévu suivant l'invention, une huile végétale en particulier, une huile lubrifiante végétale, plus particulièrement une huile lubrifiante végétale par exemple traitée par décharges électriques présentant une viscosité dynamique comprise entre 700 mPa.s et 1400 mPa.s et présentant un temps de relaxation inférieur ou égale à 200 s mesurés à 40 °C par un viscosimètre cône-plan, selon la norme ISO 2884-1.

En effet, pour certaines applications, comme par exemple dans la cosmétique, dans l'alimentaire ou encore dans les lubrifiants (par exemple moteurs), il est avantageux de disposer d'une huile végétale dont la composition est contrôlée ainsi que les propriétés physico-chimiques.

La viscosité dynamique est mesurée à l'aide d'un viscosimètre Anton Paar muni d'un système cône-plan, CP50-0.5, selon la norme ISO 2884-1 (Détermination de la viscosité au moyen de viscosimètres rotatifs). Les mesures sont obtenues sous contrainte de cisaillement de 0 à 500 s⁻¹ en prenant un point toutes les secondes, un maintien pendant une minute à 500 s⁻¹ et finalement de 500 à 0 s⁻¹ en prenant un point toutes les secondes à une température de 40 °C.

Le temps de relaxation correspond au temps nécessaire à la substance, qui présente un caractère viscoélastique, pour revenir à son état initiale lorsqu'elle est soumise à une vitesse de cisaillement de 1 s⁻¹ après avoir été soumise à une vitesse de cisaillement de 1000 s⁻¹. Une contrainte est appliquée à un échantillon de l'huile végétale et la réponse résultante de cette contrainte est suivie au cours du temps.

Selon la présente invention, le temps de relaxation de l'huile végétale a été mesuré dans un viscosimètre Anton Paar muni d'un système cône-plan (CP50-0,5) en appliquant de manière successive une vitesse de cisaillement constante de 1s⁻¹ pendant 600 s puis une vitesse de cisaillement constante à 1000s⁻¹ pendant 180 s à une température de 40 °C à l'huile végétale. La viscosité est alors mesurée au cours du temps. Après la fin de l'application d'un taux de cisaillement à 1000s⁻¹, le temps de relaxation peut être déduit en calculant le temps nécessaire pour que l'huile végétale retrouve sa viscosité d'origine avant l'application de la vitesse de cisaillement à 1000s⁻¹.

L'huile végétale selon la présente invention présente donc, comme mentionné ci-dessus une viscosité contrôlable, contrôlée et reproductible. Certaines applications dans lesquelles une onctuosité améliorée est recherchée nécessitent l'utilisation d'une huile végétale présentant une viscosité comprise entre 700 mPa.s et 1400 mPa.s comme huile de base.

Une viscosité contrôlée permet, entre autres, de prévoir la texture finale du produit qui est une caractéristique importante dans le domaine de la cosmétique ou de l'alimentaire. Par ailleurs, lorsque l'huile selon la présente invention est utilisée comme lubrifiant, sa viscosité particulière permet également de maintenir le caractère lubrifiant de l'huile par exemple lorsqu'elle est utilisée dans les moteurs où elle est soumise à des contraintes de température.

Une huile végétale présentant une gamme de viscosité et un temps de relaxation selon la présente invention est d'un grand intérêt dans le domaine des compositions, par exemple cosmétiques ou pharmaceutiques. En effet, de ce domaine, il est nécessaire de pouvoir préparer une composition comprenant une suspension en utilisant par exemple une huile végétale qui présente l'avantage de ne pas être toxique et qui possède une viscosité suffisante pour que la suspension reste stable au cours du temps et que les particules mises en suspension ne sédimentent pas. Des additifs habituellement mis en suspension peuvent être par exemple des particules de talc, poudres minérales, comme du talc, de la nacre, de métaux ou de leurs oxydes, comme par exemple d'or ou de tout métal ou d'oxyde de fer ou leur mélanges. Par comparaison, dans le cas d'une huile présentant une faible viscosité, la suspension risque de ne plus être stable au cours du temps, ce qui donne lieu à un phénomène de sédimentation et/ou d'agrégation. Par exemple, si les particules sédimentent dans le fond du récipient dans lequel se trouve la composition, celle-ci s'en trouve inutilisable. Plus particulièrement dans le domaine de la cosmétique, des huiles dans lesquelles les particules métalliques destinées à donner un effet lumière sur la peau ont sédimenté provoque de nombreux désagréments pour l'utilisateur. En effet, il est alors nécessaire d'agiter fortement le flacon d'huile pour remettre les particules en suspension. Malheureusement, l'agitation manuelle n'est pas suffisante pour remettre parfaitement en suspension de manière homogène les particules métalliques ou de nacre en suspension. Ceci provoque une inhomogénéité à l'application, ce qui ne rend pas l'utilisateur satisfait à l'utilisation de l'huile.

Le temps de relaxation inférieur ou égal à 200 s est également d'un grand intérêt. En effet, le temps de relaxation est lié aux propriétés thixotropiques de ladite huile végétale. Il a été observé que lorsque l'huile végétale selon la présente invention est soumise à une contrainte, sa viscosité va diminuer. Le temps de relaxation correspond au temps nécessaire à ladite huile, qui présente un caractère viscoélastique, pour revenir à sa viscosité initiale après arrêt de la contrainte. Concrètement, lors de la formulation d'une composition, l'huile va être mélangée (et donc soumise à une contrainte) avec par exemple du talc, de l'oxyde de fer, du nacre ou leur mélange. A cause de la contrainte, la viscosité de l'huile va diminuer, ce qui va permettre de réaliser un mélange homogène. Lorsque l'huile n'est plus mélangée, et que la contrainte s'arrête, l'huile retourne à son état initial d'huile à viscosité élevée. Elle reprend donc sa viscosité initiale après un temps de relaxation selon la présente invention. Celui-ci est inférieur ou égal à 200 secondes, ce qui est assez court pour empêcher les particules de la formulation de sédimenter et/ou de s'agréger. Le temps de relaxation de l'huile végétale selon la présente invention permet donc à la suspension de garder une certaine homogénéité. L'huile végétale selon la présente invention permet donc entre autre de réaliser des compositions dans le domaine cosmétique sous forme de suspensions. Grace aux propriétés de l'huile végétale selon la présente invention, lesdites suspensions sont homogènes et stables au court du temps.

Un autre avantage de l'huile végétale selon la présente invention réside dans le fait que certaines compositions, par exemple dans le domaine cosmétique ou pharmaceutique doivent pouvoir être versées, ce qui implique une viscosité plus faible. Cependant, une viscosité faible peut être un problème à cause par exemple du problème de sédimentation déjà mentionné. Il y a donc intérêt de disposer d'huile végétale selon la présente invention. En effet, la viscosité selon la présente invention permet d'éviter les phénomène de sédimentation comme déjà expliqué. Cependant, il peut être mal aisé de verser une huile possédant une telle viscosité. Dès lors, en soumettant l'huile à une contrainte, par exemple en la secouant ou en la mélangeant, la viscosité de celle-ci va momentanément diminuer, ce qui permet de la verser par exemple, sans perturber l'homogénéité de la suspension car les particules fines n'ont pas le temps de sédimenter durant la période de temps correspondant au temps de relaxation. Puis, après un temps de relaxation après arrêt de la contrainte, l'huile reprend sa haute viscosité initiale ce qui empêche tout phénomène de sédimentation et permet le stockage de la suspension.
De plus, l'huile végétale selon la présente invention avec sa viscosité particulièrement élevée permet de maintenir en suspension différentes tailles de particules, plus ou moins grande, sans devoir adapter l'huile qui la contient et que comme le traitement par décharge électrique est également connue pour désodoriser, c'est particulièrement adapté en cosmétique car des parfums sont typiquement ajoutés.

Une huile végétale présentant cette gamme de viscosité est également appréciée dans le domaine cosmétique ou le domaine alimentaire pour son onctuosité améliorée.

Lorsque l'on applique l'état de la technique, on se heurte d'une part au manque d'informations nécessaires à la production d'une huile de viscosité particulière. D'autre part, on n'aboutit pas à un contrôle des propriétés de l'huile car le traitement par décharges électriques de l'huile permet la réticulation (cross-linking) des molécules de l'huile qui résulte en l'augmentation de la viscosité de la substance traitée. En effet, le traitement par décharges électriques mène à la formation de triglycérides radicalaires qui peuvent alors réagir avec l'hydrogène contenu dans la cuve de traitement menant ainsi à la diminution du nombre d'insaturations dans l'huile. D'autre part, les radicaux de triglycérides peuvent également réagir avec d'autres triglycérides résultant alors en la réticulation de l'huile. Ces deux phénomènes, à savoir la diminution du nombre d'insaturations par l'hydrogénation des doubles liaisons ou la réticulation des triglycérides mènent tous deux à l'augmentation de la viscosité de l'huile liée à la réduction de la mobilité des chaînes de triglycérides. Par conséquent, le produit connu n'est pas réellement utilisable car il n'est pas stable et présente une composition aléatoire et donc difficilement reproductible. Les propriétés physico-chimiques de l'huile issue du traitement par décharges électriques de l'état de la technique sont aléatoires et difficilement contrôlées. Il n'est donc pas évident de contrôler la viscosité d'une huile lors d'un traitement par décharges électriques tel que décrit dans l'état de la technique.

Pour assurer une utilisation dans de nombreuses applications, il faut que l'huile soit viscoélastique. Malheureusement, dans les huiles connues, la réticulation est trop importante et l'huile résultante tend alors vers un état élastique pur. En effet, la réticulation des triglycérides de l'huile mène à la réduction de la mobilité des chaînes de triglycérides. Celles-ci glissent de plus en plus difficilement les unes sur les autres. De ce fait, si cette réticulation n'est pas correctement contrôlée et devient trop importante, le caractère viscoélastique de l'huile lubrifiante tend à être remplacé par un caractère élastique pur. Or selon la présente invention, une huile présentant un temps de relaxation inférieur à 200 s a été obtenue, ce qui en fait un candidat pour de nombreuses applications dans lesquelles le caractère viscoélastique de l'huile est important, notamment dans les domaines alimentaire et cosmétique ou encore dans le domaine des lubrifiants pour moteur. Par exemple dans le domaine des lubrifiants pour moteur, le temps de relaxation faible de l'huile selon la présente invention permet à l'huile de retrouver sa viscosité initiale entre deux régimes du moteur. Le temps de relaxation faible de l'huile selon la présente invention permet donc à l'huile de maintenir ses propriétés liées à sa viscosité particulière et ce malgré les contraintes auxquelles est soumise l'huile lors de son utilisation.

Un autre avantage lié au faible temps de relaxation de l'huile végétale selon la présente invention est lié au fait qu'il permet de résoudre les problèmes de lubrification liés au phénomène de « stick-slip » connu de l'homme de l'art. C'est un phénomène mécanique très courant qui se produit par exemple lorsque l'on essaye de mettre en mouvement un poids posé sur une surface et que le coefficient de frottement statique est supérieur au coefficient de frottement dynamique. Il provoque un mouvement saccadé responsable d'usures prématurées et disfonctionnements des pièces qui y sont soumis. C'est mouvement saccadés traduisent le fait que l'objet est alternativement statique et en mouvement. La thixotropie et le temps de relaxation selon la présente invention permettent de réduire les mouvements saccadés voir de les empêcher. Ceci est dû au fait que la viscosité de l'huile selon la présente invention va changer et s'adapter en fonction de la vitesse de l'objet, ce qui permet une lubrification adéquate.

Par ailleurs, l'huile végétale selon la présente invention peut être directement utilisée comme lubrifiant et ne nécessite pas un mélange avec d'autres substances végétales et/ou minérales afin d'aboutir à la viscosité souhaitée. En effet, certains documents antérieurs enseignent que pour ajuster la viscosité d'une substance lubrifiante, il est nécessaire de mélanger l'huile lubrifiante traitée par décharges électriques avec d'autres substances végétales et/ou minérales. L'ajout d'une substance minérale peut conduire à l'obtention d'un produit présentant une certaine toxicité. En effet, les huiles minérales peuvent être issues de la distillation du pétrole, peuvent dès lors contenir des hydrocarbures aromatiques présentant une toxicité sévère, notamment pour l'homme.

Le contrôle des propriétés physico-chimiques de l'huile végétale selon la présente invention a comme avantage de pouvoir utiliser cette dernière pour de nombreuses applications pare exemple dans le secteur automobile, le secteur cosmétique, le secteur alimentaire...

De préférence, le temps de de relaxation de l'huile végétale selon la présente invention est inférieur ou égale à 190 s, avantageusement inférieur ou égale à 180 s, préférentiellement inférieur ou égale à 170 s, de préférence inférieur ou égal à 160 s mesuré à 40 °C par un viscosimètre cône-plan, selon la norme ISO 2884-1.

De manière encore plus avantageuse, le temps de de relaxation de l'huile végétale selon la présente invention est inférieur ou égal à 50 s, de préférence, inférieur ou égal à 10s.

Avantageusement, l'huile végétale selon la présente invention est caractérisée en ce qu'elle présente une thixotropie comprise entre 5 % et 60 %, de préférence entre 5 % et 30 % de préférence entre 10 % et 20 % de la viscosité dynamique.

La thixotropie est une mesure de la variation de la viscosité lors que l'huile est soumise à une contrainte. Il s'agit d'une propriété physique d'un fluide dont la viscosité varie au cours du temps lorsque le fluide est soumis à une contrainte constante (ou un gradient de vitesse). La thixotropie est un phénomène physique qui résulte de la non-instantanéité des processus de destruction et de réédification de la structure microscopique par agitation et par repos d'une substance telle que l'huile. Le comportement thixotrope est défini comme un comportement dépendant du temps et est correctement déterminé lorsque l'on considère la décomposition et la régénération de la substance testée sous contrainte de cisaillement constante dans un intervalle de temps défini.

Selon la présente invention, la thixotropie de l'huile est représentée par la variation de la viscosité entre l'état initial et l'état déstructuré de l'huile.

Selon la présente invention, la thixotropie de l'huile végétale a été mesurée lors d'un test réalisé sous contrainte de cisaillement constante de 1000 s⁻¹ à une température de 40 °C à l'aide d'un viscosimètre Anton Paar muni d'un système cône-plan, CP50-0.5.

De préférence, l'huile végétale selon la présente invention est caractérisée en ce qu'elle présente un indice d'iode compris entre 50 et 200 mg, de préférence entre 100 et 180 mg.

L'indice d'iode d'un lipide est la masse de diiode (I₂) capable de se fixer sur les insaturations des triglycérides contenus dans cent grammes de matière grasse.

Selon la présente invention, l'indice d'iode a été mesuré par la méthode de Wijs qui consiste à faire réagir un excès connu de monochlorure d'iode (ICl) sur le corps gras à analyser, à savoir l'huile végétale. Le monochlorure d'iode se fixe sur les doubles liaisons de l'échantillon analysé et l'excès de réactif reste en solution. De l'iodure de potassium est alors ajouté en excès à cette solution provoquant ainsi le retour du cation I⁺ en excès à l'état moléculaire I₂ dissous. Le diiode peut alors être dosé par une solution de concentration molaire connue de thiosulfate de sodium, en présence d'empois d'amidon.

L'huile végétale selon l'invention présente avantageusement en outre, une masse molaire comprise entre 6000 et 20000 g/mol. Cette masse molaire est exprimée en équivalent polystyrène, comme déterminé par chromatographie d'exclusion stérique (Agilent) fonctionnant à un débit de 1 mL.min⁻¹ à une température de 30 °C. Les échantillons sont solubilisés dans le chloroforme à 1 mg.mL⁻¹ et sont fractionnés par passage à travers 2 colonnes PL GEL MIX-D 10. Les colonnes ont été préalablement étalonnées en utilisant des polystyrènes de faible dispersité de masse molaire comprise entre 500 et 10⁶ g.mol⁻¹. La détection est assurée par un détecteur d'indice de réfraction (Agilent DRI).

L'allongement de chaînes des triglycérides contenus dans l'huile résulte également en l'augmentation de la masse moléculaire de l'huile traitée.

De plus, dans une forme de réalisation particulière de l'huile végétale selon l'invention est une huile végétale choisie dans le groupe constitué d'une huile de colza, d'une huile de lin, d'une huile d'argan et leurs mélanges.

De préférence, il s'agit d'huiles végétales insaturées présentant avant traitement un indice d'iode compris entre 100 et 180 mg.

D'autres formes de réalisation de l'huile végétale suivant l'invention sont indiquées dans les revendications annexées.

La présente invention se rapporte également à l'utilisation de l'huile végétale selon la présente invention dans le domaine cosmétique.

Avantageusement, l'huile végétale selon la présente invention est utilisée dans le domaine alimentaire.

D'autres utilisations de l'huile végétale suivant l'invention sont indiquées dans les revendications annexées.

De manière générale pour obtenir une huile végétale selon la présente invention, une huile végétale est traitée par plasma. Tout d'abord, environ 2 litres d'huile végétale sont placés dans une cuve. Cette dernière est une enceinte circulaire contenant une pluralité d'électrodes connectées à une source de courant alternatif et une pluralité d'électrodes de masse connectées à la terre. Ces électrodes sont des disques en aluminium de 25 cm de diamètre et d'une épaisseur de 2 mm. Entre ces électrodes sont placées des disques en pyrex d'un diamètre de 28 cm et d'une épaisseur de 5 mm. Sauf indication contraire, l'enceinte est ensuite remplie d'hydrogène à une pression de 180 Torr. La cuve est ensuite mise en rotation à une vitesse de 5 tours par minutes, et sauf indication contraire, une tension de 2900 V est appliquée aux électrodes. Il reste ensuite à appliquer une tension et une fréquence précises. Ces fréquences sont choisies de manière à ce que le courant de décharge soit maximal et stable. Dans ce cas-ci par exemple, des fréquences de 35 kHz ou 66 kHz peuvent être utilisées.

En outre, il a été observé qu'en fonction de la fréquence utilisée et du temps de traitement, il devient possible de contrôler le temps de relaxation et la viscosité des huiles végétales traitées. Plus le temps de traitement sera long, plus la viscosité de l'huile traitée sera élevée. Il a été découvert que la fréquence utilisée permet aussi d'influencer le temps de relaxation. En effet, par exemple à une fréquence de 66kHz, le temps de relaxation sera inférieur ou égal à 200s. A une fréquence de 35kHz, le temps de relaxation sera encore inférieur à 100s, voir inférieur à 10s. Un traitement à basse fréquence nécessite cependant un tant de traitement plus long qu'à haute fréquence pour atteindre une valeur de viscosité d'huile similaire.
La figure 1 est un graph décrivant l'évolution de la viscosité de l'huile en fonction du temps de traitement et en fonction de la fréquence utilisée pour une huile de colza et une huile de lin.
La figure 2 montre une photographie d'une dispersion de 0,5% en poids d'oxyde de fer dans une huile de colza selon la présente invention après 15 jours de repos.

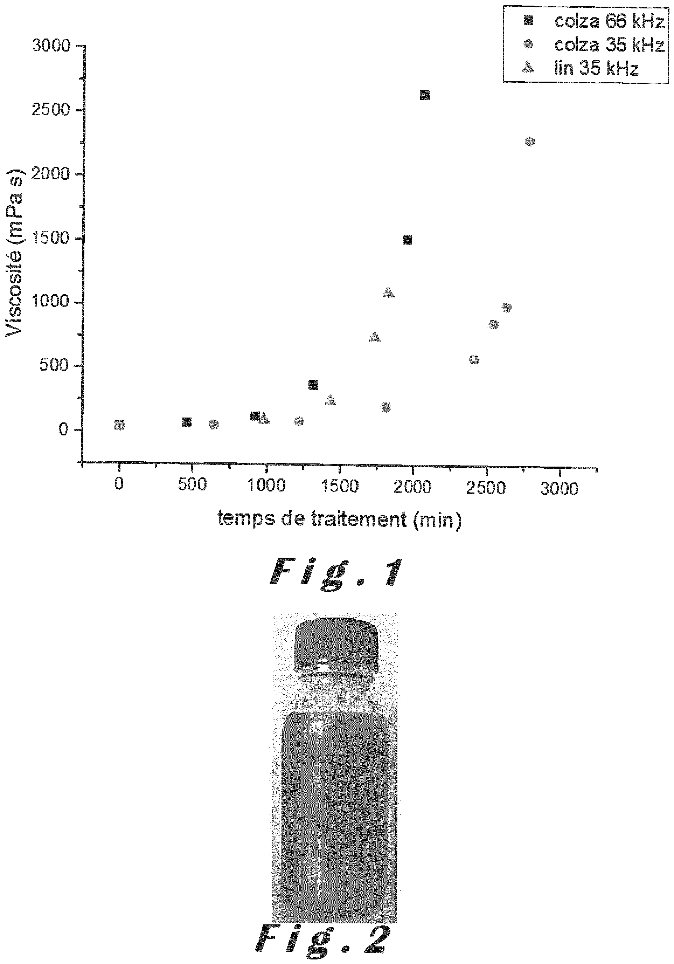

La figure 1 montre que la viscosité des huiles végétale traitées peut être contrôlée en fonction du temps de traitement et de la fréquence appliquée. De manière générale, il apparait que plus le temps de traitement est long, plus la viscosité de l'huile traitée augmente. De plus, il apparait clairement dans la figure 1 que le traitement à 35kHz nécessite un temps de traitement plus long que lorsque le traitement est réalisé à 66kHz pour atteindre une valeur de viscosité d'huile similaire. Il est dès lors possible de déduire qu'au plus la fréquence sera élevée, au plus court devra être le temps de traitement pour obtenir une valeur de viscosité désirée.

### Exemples

Une huile végétale a été traitée dans une cuve permettant d'appliquer un plasma à l'huile végétale sus mentionnée. Cette cuve est une enceinte circulaire contenant une pluralité d'électrodes connectées à une source de courant alternatif et une pluralité d'électrodes de masse connectées à la terre. Ces électrodes sont des disques en Aluminium de 25 cm de diamètre et d'une épaisseur de 2 mm. Entre ces électrodes sont placées des disques en pyrex d'un diamètre de 28 cm et d'une épaisseur de 5 mm.

2 litres d'huile sont placés dans la cuve et celle-ci est mise en dépression jusqu'à atteindre un vide de 10⁻² mbar. De l'hydrogène est alors introduit dans l'enceinte pour atteindre une pression de 180 Torr, sauf indication contraire.

La cuve est mise en rotation autour d'un arbre de rotation à une vitesse de 5 tours par minute.

Sauf indication contraire, une tension de 2900 V est appliquée aux électrodes, ce qui correspond à un courant de décharge de 2,5 A et à une fréquence précisée dans les exemples ci-dessous.

### Exemple 1

Le traitement décrit ci-dessus a été réalisé à une fréquence de 35 kHz sur une huile de Colza de la marque AVENO et répété pour différents temps de traitement prédéterminés afin d'obtenir des huiles végétales traitées aussi appelées lubrifiantes de propriétés physico-chimiques différentes. Ces huiles végétales obtenues après différents temps de traitement présentent une structure visuellement homogène, sans agrégats ni agglomérats. Ces huiles ont été analysées et présentent les caractéristiques reprises dans le tableau 1.

**Tableau 1**

| **Temps de traitement (min)** | **Insaturations - indice d'iode (mg)** | **Disparition de double liaison (%)** | **Masse moléculaire (g/mol)** | **Viscosité à 40° (mPa s)** | **Thixotropie (mPa s)** | **Temps de relaxation (s)** |
|---|---|---|---|---|---|---|
| 2540,0 | 77,9 | 27,8 | 21440,0 | 865,0 | 248,0 | <10 |
| 2628,0 | 77,9 | 25,3 | 24900,0 | 1150,0 | 301,0 | <10 |

### Exemple 2

Le traitement décrit ci-dessus a été réalisé à une fréquence de 66 kHz sur une huile de lin durant 1315 minutes afin d'obtenir une huile végétale traitée aussi appelée lubrifiante de propriétés physico-chimiques contrôlées. Cette huile végétale obtenue après traitement présente une structure visuellement homogène, sans agrégats ni agglomérats. Cette huile a été analysée et présente les caractéristiques reprises dans le tableau 2.

**Tableau 2**

| **Temps de traitement (min)** | **Insaturations - indice d'iode (mg)** | **Disparition de double liaison (%)** | **Masse moléculaire (g/mol)** | **Viscosité à 40° (mPa s)** | **Thixotropie (mPa s)** | **Temps de relaxation (s)** |
|---|---|---|---|---|---|---|
| 1315 | 130,4 | 26,5 | 19220 | 1260 | 500 | 170 |

### Exemple 3

Le traitement décrit ci-dessus a été réalisé à une fréquence de 35 kHz sur une huile de lin durant 1730 minutes afin d'obtenir une huile végétale traitée aussi appelée lubrifiante de propriétés physico-chimiques contrôlées. Cette huile végétale obtenue après traitement présente une structure visuellement homogène, sans agrégats ni agglomérats. Cette huile a été analysée et présente les caractéristiques reprises dans le tableau 3.

**Tableau 3**

| **Temps de traitement (min)** | **Insaturations - indice d'iode (mg)** | **Disparition de double liaison (%)** | **Masse moléculaire (g/mol)** | **Viscosité à 40° (mPa s)** | **Thixotropie (mPa s)** | **Temps de relaxation (s)** |
|---|---|---|---|---|---|---|
| 1730,0 | 142,7 | 27,8 | 19730,0 | 750,0 | 210,0 | <10 |

Ces exemples permettent de mettre en évidence que sur toute la gamme de viscosité de l'huile selon la présente invention, le temps de relaxation est inférieur à 200 s. De plus, cette huile présente une thixotropie de maximum 30% de la viscosité. On peut donc en conclure que l'huile végétale selon la présente invention possède une viscosité améliorée et contrôlée tout en présentant un caractère viscoélastique et thixotrope amélioré.

### Exemple 4

Le traitement décrit ci-dessus a été réalisé à une tension de 2640 V et une fréquence de 66 kHz sur une huile de Colza de la marque AVENO durant 1860 minutes afin d'obtenir une huile végétale traitée aussi appelée lubrifiante de propriétés physico-chimiques contrôlées. La pression en hydrogène était de 150 Torr. Cette huile végétale obtenue après traitement présente une structure visuellement homogène, sans agrégats ni agglomérats. Cette huile a été analysée et présente les caractéristiques reprises dans le tableau 4.

**Tableau 4**

| **Temps de traitement (min)** | **Viscosité à 40 ° (mPa s)** | **Temps de relaxation (s)** |
|---|---|---|
| 1860 | 1090,0 | <200 |

Ces exemples permettent de mettre en évidence que l'huile végétale traitée a une viscosité comprise entre 700 mPa.s et 1400 mPa.s et un temps de relaxation est inférieur à 200 s. De plus, cette huile présente une thixotropie de 30% de la viscosité. On peut donc en conclure que l'huile végétale selon la présente invention possède une viscosité améliorée et contrôlée tout en présentant un caractère viscoélastique et thixotrope amélioré.

### Mise en suspension

60 g d'huile de colza traitée selon l'exemple 4 a été mélangée avec de l'oxyde de fer de manière à ce que le pourcentage en poids en oxyde de fer atteigne 0,5%. Le mélange a été agité et porté à la température de 60°C grâce à un barreau magnétique et un agitateur magnétique de type IKA RET Control. Une fois la température atteinte, l'agitation magnétique a été maintenue pendant une dizaine de minutes à la vitesse de mélange de 250 tr/ min afin d'assurer une dispersion homogène. Le mélange a été versé dans une bouteille en verre de 50 ml directement après le mélange.

Le mélange a été laissé à reposer pendant 15 jours. Après 15 jours, la majeure partie de l'oxyde de fer n'avait pas sédimentée de manière visible. La figure 2 est une photographie de cette suspension après avoir été reposée pendant 15 jours. On peut clairement observer que cette suspension est encore stable.

### Exemple comparatif 1.-

Afin de mettre en évidence l'effet avantageux que ces huiles procures dans des applications telle que la cosmétique, un test comparatif de mise en suspension a été réalisé par rapport à une huile de colza commerciale n'ayant pas subi de traitement.

### Mise en suspension dans une huile de colza commerciale

60 g d'huile de colza commerciale Alvenat a été mélangée avec de l'oxyde de fer de manière à ce que le pourcentage en poids en oxyde de fer atteigne 0,5%. Le mélange a été agité et porté à la température de 60°C grâce à un barreau magnétique et un agitateur magnétique de type IKA RET Control. Une fois la température atteinte, l'agitation magnétique a été maintenue pendant une dizaine de minutes à la vitesse de mélange de 250 tr/ min afin d'assurer une dispersion homogène. Le mélange a été versé dans une bouteille en verre de 50 ml directement après le mélange.

Le mélange a été laissé à reposer. Déjà après 15 jours, l'oxyde de fer avait sédimenté de manière visible.

En consultant la figure 2, il apparait clairement que des suspensions dans l'huile végétale selon la présente invention sont stables au cours du temps, ce qui n'est pas le cas des suspensions dans les huiles de colza commerciales non traitées Alvenat.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Huile végétale, en particulier une huile lubrifiante végétale, par exemple traitée par décharges électriques **caractérisée en ce qu'**elle présente une viscosité dynamique comprise entre 700 mPa.s et 1400 mPa.s et un temps de relaxation inférieur ou égal à 200 s, mesurés à 40 °C par un viscosimètre cône-plan, selon la norme ISO 2884-1, le temps de relaxation étant mesuré en appliquant de manière successive une vitesse de cisaillement constante de 1s⁻¹ pendant 600s puis une vitesse de cisaillement constante à 1000s⁻¹ pendant 180s à l'huile végétale.

2. Huile végétale selon la revendication 1 **caractérisée en ce qu'**elle présente un temps de relaxation inférieur ou égal à 50 s, de préférence inférieur ou égal à 10 s.

3. Huile végétale selon la revendication 1 **caractérisée en ce qu'**elle présente une thixotropie comprise entre 5% et 60%, de préférence entre 5% et 30%, de préférence entre 10% et 20 % de la viscosité dynamique.

4. Huile végétale selon l'une quelconque des revendications 1 à 3 **caractérisée en ce qu'**elle présente un indice d'iode compris entre 50 et 200 mg, de préférence entre 100 et 150 mg.

5. Huile végétale selon l'une quelconque des revendications 1 à 4 **caractérisée en ce qu'**elle présente une masse molaire comprise entre 6000 et 20000 g/mol.

6. Huile végétale selon l'une quelconque des revendications 1 à 5 **caractérisée en ce qu'**elle est une huile végétale choisie dans le groupe constitué d'une huile de colza, d'une huile de lin, d'une huile d'argan et de leurs mélanges.

7. Utilisation de l'huile végétale selon l'une quelconque des revendications précédentes en tant que lubrifiant pour moteur.

8. Utilisation de l'huile végétale selon l'une quelconque des revendications 1 à 6 dans le domaine cosmétique.

9. Utilisation de l'huile végétale selon l'une quelconque des revendications 1 à 6 dans le domaine alimentaire.

## Patentansprüche

1. Pflanzenöl, insbesondere ein pflanzliches Schmieröl, beispielsweise durch elektrische Entladungen behandelt, **dadurch gekennzeichnet, dass** es eine dynamische Viskosität zwischen 700 mPa·s und 1400 mPa·s und eine Relaxationszeit von weniger als oder gleich 200 s aufweist, gemessen bei 40 °C mit einem Kegel-Ebenen-Viskosimeter gemäß der ISO-Norm 2884-1, wobei die Relaxationszeit gemessen wird, indem nacheinander eine konstante Scher-geschwindigkeit von 1 s⁻¹ für 600 s und dann eine konstante Schergeschwindigkeit von 1000 s⁻¹ für 180 s auf das Pflanzenöl angewendet wird.

2. Pflanzenöl nach Anspruch 1, **dadurch gekennzeichnet, dass** es eine Relaxationszeit von weniger als oder gleich 50 s, bevorzugt weniger als oder gleich 10 s, aufweist.

3. Pflanzenöl nach Anspruch 1, **dadurch gekennzeichnet, dass** es eine Thixotropie zwischen 5 % und 60 %, bevorzugt zwischen 5 % und 30 %, bevorzugt zwischen 10 % und 20 % der dynamischen Viskosität aufweist.

4. Pflanzenöl nach einem der Ansprüche 1 bis 3, **dadurch gekennzeichnet, dass** es einen Jodindex zwischen 50 und 200 mg, bevorzugt zwischen 100 und 150 mg aufweist.

5. Pflanzenöl nach einem der Ansprüche 1 bis 4, **dadurch gekennzeichnet, dass** es eine Molmasse zwischen 6000 und 20000 g/mol aufweist.

6. Pflanzenöl nach einem der Ansprüche 1 bis 5, **dadurch gekennzeichnet, dass** es sich um ein Pflanzenöl handelt, ausgewählt aus der Gruppe bestehend aus Rapsöl, Leinöl, Arganöl und Gemischen davon.

7. Verwendung des Pflanzenöls nach einem der vorhergehenden Ansprüche als Motorschmiermittel.

8. Verwendung des Pflanzenöls nach einem der Ansprüche 1 bis 6 im kosmetischen Bereich.

9. Verwendung des Pflanzenöls nach einem der Ansprüche 1 bis 6 im Lebensmittelbereich.

## Claims

1. Vegetable oil, in particular a lubricating vegetable oil, treated, for example, by electrical discharges, **characterised in that** is has a dynamic viscosity of between 700mPa.s and 1400mPa.s and a relaxation time of less than or equal to 200s, measured at 40°C by a cone and plate viscometer, according to standard ISO 2884-1, the relaxation time being measured by successively applying a constant shear rate of 1s⁻¹ for 600s, then a constant shear rate of 1000s⁻¹ for 180s to the vegetable oil.

2. Vegetable oil according to claim 1, **characterised in that** it has a relaxation time of less than or equal to 50s, preferably less than or equal to 10s.

3. Vegetable oil according to claim 1, **characterised in that** it has a thixotropy of between 5% and 60%, preferably between 5% and 30%, preferably between 10% and 20% of the dynamic viscosity.

4. Vegetable oil according to any one of claims 1 to 3, **characterised in that** it has an iodine value of between 50 and 200mg, preferably between 100 and 150mg.

5. Vegetable oil according to any one of claims 1 to 4, **characterised in that** it has a molar mass of between 6000 and 20000g/mol.

6. Vegetable oil according to any one of claims 1 to 5, **characterised in that** it is a vegetable oil chosen from the group consisting of rapeseed oil, linseed oil, argan oil and mixtures thereof.

7. Use of the vegetable oil according to any one of the preceding claims as engine lubricant.

8. Use of the vegetable oil according to any one of claims 1 to 6 in the cosmetics industry.

9. Use of the vegetable oil according to any one of claims 1 to 6 in the food industry.
